Europäisches Patentamt

European Patent Office

Office européen des brevets

(11) Publication number: **0 195 773**
**B1**

# EUROPEAN PATENT SPECIFICATION

(45) Date of publication of patent specification: **08.11.89**

(51) Int. Cl.⁴: **C 12 P 19/06**

(21) Application number: **84903493.9**

(22) Date of filing: **19.09.84**

(86) International application number:
**PCT/GB84/00319**

(87) International publication number:
**WO 86/01830 27.03.86 Gazette 86/07**

(54) CONTINUOUS PRODUCTION OF BACTERIAL POLYSACCHARIDE.

(43) Date of publication of application:
**01.10.86 Bulletin 86/40**

(45) Publication of the grant of the patent:
**08.11.89 Bulletin 89/45**

(84) Designated Contracting States:
**CH DE FR GB LI NL SE**

(56) References cited:
**EP-A-0 045 569**
**FR-A-2 328 770**
**US-A-4 374 929**
**US-A-4 400 467**

**Chemical Abstracts, vol. 80, no. 13, 1 april 1984
(Columbus OHIO; US)**

(73) Proprietor: **The Secretary of State for Defence in
Her Britannic Majesty's Government of the
United Kingdom of Great Britain and
Northern Ireland Whitehall
London SW1A 2HB (GB)**

(72) Inventor: **ELLWOOD, Derek, Clifford
Upper Close Winterbourne Stoke
Nr Salisbury Wiltshire SP3 4SW (GB)**
Inventor: **EVANS, Charles, Gervase, Thorngate
81 Castle Road
Salisbury Wiltshire SP1 3RW (GB)**
Inventor: **YEO, Richard, Grenville
Chanterelle Roman Road East Winterslow
Salisbury Wiltshire SP5 1QD (GB)**

(74) Representative: **Beckham, Robert William et al
Procurement Executive Ministry of Defence
Patents 1A(4), Room 2014
Empress State Building Lillie Road London SW6
1TR (GB)**

# EP 0 195 773 B1

**Description**

It is well known that certain bacteria of the genus *Xanthomonas*, notably the *Xanthomonas campestris* group, when cultured under suitable conditions are capable of producing hetero-polysaccharide, commonly termed "Xanthan gum", which are useful as thickeners and emulsifiers in a variety of applications including foodstuffs and drilling muds. At present the bacteria are generally grown in batch culture, typically for 48 to 72 hours, in conventional complex culture media containing nutrients such as corn starch (US Patent 3,455,786), soy peptone (US Patents 3,391,060 and 3,391,061) distillers solubles (French Patent 2,251,620) or "Stimuflav" (US Patent 3,020,206).

Whilst batch production of xanthan gum requires at least 48 to 72 hours to achieve maximum yield, similar yields per unit volume of culture may be obtained in only 12 to 50 hours by continuous methods. The desirability of producing xanthan gum by continuous methods was therefore recognised at an early stage (US 3,328,262, Lindblom and Patton). However successive attempts to produce an economically viable system (US 3,485,719, Rogovin; Silman and Rogovin, Biotechnol, Bioeng, 1970, 12, 75 and 1972, 14, 23) were unsuccessful because the bacteria (*Xanthomonas*), under the conditions employed, developed strains that did not produce polysaccharide. (It should be noted that to compete with a batch system, a continuous culture should proceed for at least 10 turnovers of culture medium (culture turnover = Q = duration of culture × dilution rate), and preferably for much longer than that).

The problems of *Xanthomonas* strain variation (to give non-polysaccharide producing strains) was overcome in our own UK 1512536 by the use of a "chemically defined medium". In that patent we defined a "chemically defined medium" as a "culture medium wherein nutrients other than carbon are provided as inorganic salts or as single organic compounds of known molecular structure". We went on to say, however, that "normally the only organic component of the chemically defined medium will be a conventional carbon source ..." and we preferred the use of ammonium salts as the nitrogen source.

EP—A—0045569 describes a process in which *Xanthomonas* bacteria are cultured in a medium in which the principal nitrogen source is ammonium chloride, with glutamate, glutamic acid or asparagine among other supplementary nitrogen sources. The process described in EP—A—0045569 is a continuous process.

We have now discovered that in some circumstances and under certain carefully controlled conditions the use of selected organic compounds of known molecular structure as the nitrogen source in such a chemically defined medium can offer a number of advantages over the previously preferred use of ammonium salts. In particular we have found that the output of polysaccharide can be significantly increased at higher dilution rates if the appropriate organic compound is chosen as the nitrogen source.

It is therefore one object of the present invention to provide a process for the continuous production of bacterial polysaccharide in which the output of polysaccharide at low mean residence times is higher than the outputs obtained using inorganic nitrogen sources in chemically defined media at the same low mean residence times.

Other objects and advantages of the present process will become apparent from the following detailed description thereof.

Thus according to the present invention there is provided a process for the production of polysaccharide by the continuous culture of polysaccharide producing bacteria of the genus *Xanthomonas* wherein the bacteria are grown in a single stage culture in a chemically defined culture medium, said medium having a growth limiting nutrilite and consisting of, in known ratio, a carbon source at a concentration of at least 10 gm litre$^{-1}$ (calculated as elemental carbon), a nitrogen source in the form of a single organic compound of known molecular structure and sources of phosphorus, sulphur, magnesium, potassium and other essential elements in the form of inorganic salts characterised in that the nitrogen source is the limiting nutrilite and is selected from the group consisting of glutamic acid, asparagine, a glutamate salt and an asparagine salt and further characterised in that the culture has a mean residence time in the culture vessel of 16⅔ hr or less.

The invention is especially applicable to polysaccharide producing *Xanthomonas* bacteria falling within the "campestris group" as defined by Bergey's Manual of Determinative Bacteriology, 7th Edition. Bacteria of the species *Xanthomonas campestris*, such as the publicly available strains ATCC 13951, ATCC 31600, ATCC 31601, ATCC 31602 and NRRL B—1459, have been found suitable, as have bacteria of the species *Xanthomonas juglandis*, such as the publicly available strains ATCC 11329 and NCPPB 362, 411, 412, 413, 414, 415, 1447, 1659.

The culture medium used in the process of the present invention preferably contains a polyhydric alcohol, for example glycerol or a carbohydrate, such as glucose, sucrose, fructose or starch, as its carbon source. For the production of useful yields, a high concentration of carbon source is required and the total concentration of available carbon should be at least 10 gm/litre (calculated as elemental carbon). Higher concentrations especially between 15 and 24 gms/liter may often be preferred but very high concentrations may interfere with the process due to excessive increases in viscosity of the culture broth.

The nitrogen source is glutamic acid, asparagine or salts of these amino acids. Of these, glutamic acid and glutamate salts are particularly preferred. Even though the nitrogen source is the growth limiting nutrilite in the present culture medium, a concentration (calculated as elemental nitrogen) of at least 0.7 gm litre$^{-1}$ is preferred in order to obtain the higher levels of polysaccharide production that would be required in most industrial plants.

The sources of phosphorus, sulphur, magnesium, potassium and other elements essential for cell growth are inorganic salts such as phosphates, sulphates, halides, oxides and borates. In each case the concentration of the nutrilite must be at a level high enough to ensure that nitrogen remains the limiting nutrilite.

It is also important that the present culture medium (the one supplied to the culture vessel) consists of a known ratio of the known chemical ingredients. This is generally achieved by dissolving known amounts of these ingredients in a suitable, generally aqueous, solvent.

The present continuous process generally comprises four stages:

(a) the introduction of the culture of polysaccharide producing bacteria of the genus *Xanthomonas* to a culture vessel,

(b) the continuous supply of the culture medium, at a controlled rate to the culture vessel, when bacterial growth has become established,

(c) the maintenance of conditions of aeration, pH and temperature within the culture vessel to promote the simultaneous growth of bacteria and production of polysaccharide, and

(d) the continuous withdrawal of the resulting polysaccharide containing culture broth from the culture vessel at substantially the same rate as the culture medium is supplied to the culture vessel.

Prior to the introduction of the bacteria to the culture vessel a *Xanthomonas* seed culture is produced. This may be grown in either a chemically defined or a complex medium.

At first sight this may seem slightly anomalous, since it may mean that a complex medium is initially introduced to the culture vessel in the manner of the prior art. However, unlike the prior art processes in which complex components are present in the culture vessel throughout the bacterial fermentation and thereby lead to *Xanthomonas* strain variation, the present continuous process, in which culture medium is continually added to and removed from the culture vessel, ensures that the concentration of complex components in the culture vessel will be reduced to ineffective levels in a short time.

The culture conditions, apart from the culture medium, should be within the conventional ranges used for *Xanthomonas* culture. The pH of the culture should normally be between 6.0 and 8.0 and preferably between 6.5 and 7.5 for optimum polysaccharide production. The pH may be maintained at this level by controlled addition of base and/or acid using a pH controller. Alternatively or additionally, pH control may be achieved by buffering the culture medium with a conventional buffer such as sodium dihydrogen phosphate, which may also act as a phosphorus source. The temperature is less critical, but will generally be between 20° and 40°C. Temperatures within the range 25° to 35°C are generally preferred.

As further understood in the art, culture processes in accordance with the present invention will normally be conducted under aerobic conditions achieved by a flow of oxygen or air through the culture medium, generally assisted by stirring. Air flows of about 1 vol air per volume of culture each minute are generally suitable. Oxygen flow rates may, of course, be lower and the use of oxygen may therefore be preferred in very viscous (i.e. high yielding) media.

Although reasonable polysaccharide production may be achieved using mean residence times of the medium in the culture vessel (volume divided by flow rate = the reciprocal of dilution rate) as low as 8 hours, for high polysaccharide yields the mean residence time should be at least 9 hours. Optimum mean residence times will generally be between 9 and 16⅔ hours (dilution rates between 0.111 and 0.06).

It is a major advantage of the present processes, when compared with processes of the type described in UK 1512536 that, all other things being equal, the output of polysaccharide at a given mean residence time below 16⅔ hr, using glutamic acid, asparagine or salts thereof as the nitrogen source is significantly higher than the output of polysaccharide at the same mean residence time but with an inorganic salt, particularly ammonium chloride, as the nitrogen source. This output increase may have a profound effect on the cost of polysaccharide when that product is produced continuously on an industrial scale.

A further advantage of a chemically-defined medium is that it facilitates control of the growth-limiting substrate or nutrilite (also called the limiting nutrient). The rate of cell growth in the culture, in otherwise optimum conditions, will depend on the available concentration of the elements essential for growth. In practice, these concentrations will never be completely in balance and hence one element, or nutrilite, will be present at a concentration lss than proportionate to the others in relation to the requirements of the organism. This element is termed the limiting nutrilite.

The limiting nutrilite may be selected by analysing the elemental composition of the bacterial cells and arranging that elements other than the limiting nutrilite are present in the culture medium in considerably greater proportions than those found in the analysis. That growth is limited by the chosen nutrilite can be confirmed by substantially increasing (e.g. doubling) its concentration in the medium which should cause an increase in cell concentration. (The design of such chemically defined media was described by Evans, Herbert & Tempest in Methods in Microbiology, Vol. 2, pp. 210—313, edited by Norris & Ribbons, Academic Press 1970).

When inorganic nitrogen sources were employed in the continuous fermentation of *Xanthomonas* in chemically defined media, see UK 1512536, sulphur or phosphorus limitation was preferred. Surprisingly the present inventors have found that when glutamic acid, asparagine or salts thereof are employed as the nitrogen source, nitrogen limitation affords by far the best yields of polysaccharide as well as giving a superior product in terms of polymeric properties.

The xanthan gum may be separated from the culture by any convenient method known in the art. Alternatively the whole culture may be used with or without previously killing the bacteria, depending on the intended use.

Specific processes in accordance with the present invention will now be described by way of example. These *Xanthomonas* strains described are publicly available on application to the appropriate depository subject to national plant health regulations.

Crude polysaccharide yield was measured by precipitating total precipitatable material from the culture broth by the addition of three volumes of acetone. Apparent viscosities of culture broths were measured, over a range of shear rates, in a Wells-Brookfield "cone and Plate" viscometer, model HBT, at a temperature of 25°C using the 0.8° cone and 0.5 ml samples. The consistency index — The apparently viscosity at no shear and known as the K value — was determined by extrapolation from a log — log plot of apparent viscosity against shear rate.

Polysaccharide for analysis was obtained by diluted one part of culture broth with nine parts of deionised water and centrifuging at 9,000 g for 90 minutes to remove bacterial cells; the polysaccharide was then reprecipitated with three volumes of acetone. The samples so produced contained no detectable free glucose.

In all of the following examples the experiments were deliberately terminated after the stated period for experimental convenience and not because of contamination or any reduction in yield.

Example 1

A single colony of *Xanthomonas campestris* ATCC 13951 taken from a 24 hour culture plate (a mixture of a tryptic hydrolysate of casein and an enzymic digest of soya bean meal sold as Tryptone Soya Agar by Oxoid Ltd) was inoculated into a 2 litre conical flask containing 200 ml of a sterile medium composed of 3% Tryptone Soya Broth (Oxoid Ltd), 1% glucose, pH 7.0. This seed culture was incubated with aeration by shaking for 2 days at 30°C and then transformed to a 2½ litre continuous culture vessel (similar to that described by Evans *et al*, Methods in Microbiology, Vol. 2, pp. 310—313, 1970) containing 2 litres of a culture medium composed of

| | |
|---|---|
| Sodium Glutamate | — 9.35 gm/litre, equivalent to 700 mg/litre of nitrogen |
| Sodium Dihydrogen Phosphate | — 2.4 gm/litre, equivalent to 620 mg/litre of phosphorus |
| Sodium Sulphate | — 1.42 gm/litre, equivalent to 320 mg/litre of sulphur |
| Potassium Chloride | — 0.381 gm/litre, equivalent to 200 mg/litre of potassium |
| Magnesium Oxide | — 0.05 gm/litre equivalent to 30 mg/litre of magnesium |
| Glucose | — 60 gm/litre, equivalent to 24 gm/litre of carbon |

together with traces of various metals such as zinc, copper, cobalt, iron, manganese and molybdenum. Air was supplied at lvol. air/vol.culture/min. and the culture was stirred at 1000 rpm. The culture was allowed to grow batchwise for 48 hours at 30°C and pH 6.8 and then flow of the same medium to give a mean residence time of 30 hours (dilution rate 0.033 per hour) was started. The culture was maintained at 30°C and pH 6.8 throughout. When it became apparent, after 259 hours, that the culture was growing successfully the dilution rate was increased to 0.05 for 151 hours and then varied between 0.06 and 0.107 for the remainder of the experiment. Results are shown in Table 1.

TABLE 1

| Age (hr) | Dilution Rate $(hr^{-1})$ | Polysaccharide (gm/litre) | Viscosity (CPS) |
|---|---|---|---|
| 162 | 0.033 | 30.6 | 34,000 |
| 258 | 0.033 | 30.7 | 30,000 |
| 402 | 0.05 | 28.7 | 27,000 |
| 426 | 0.06 | 30.8 | 28,500 |
| 498 | 0.06 | 31.7 | 28,500 |
| 594 | 0.06 | 35.0 | 38,500 |
| 1096 | 0.06 | 36.0 | 36,000 |
| 1332 | 0.08 | 34.2 | 31,000 |
| 1500 | 0.09 | 31.6 | 31,500 |
| 1596 | 0.107 | 26.9 | 24,500 |
| 2028 | 0.10 | 29.8 | 26,000 |

Example 2

The process of Example 1 was repeated except that the dilution rate was maintained at 0.066 throughout the experiment. The results of the experiment, which was deliberately terminated after 790 hr, are shown in Table 2.

Table 2

| Age (hr) | Dilution Rate $(hr^{-1})$ | Polysaccharide (gm/litre) | Viscosity (CPS) |
|---|---|---|---|
| 115 | 0.065 | 28.2 | 18,500 |
| 259 | 0.066 | 29.2 | 20,000 |
| 331 | 0.066 | 29.5 | 22,500 |
| 499 | 0.066 | 29.6 | 21,500 |
| 619 | 0.066 | 26.0 | 21,500 |
| 787 | 0.066 | 27.8 | 21,500 |

Example 3

The procedure of example 1 was repeated except that the sodium glutamate was replaced by 3.3 gm/ litre of asparagine (700 mg/litre of nitrogen). The dilution rate was 0.042 $hr^{-1}$ for the first 598 hr, after which it was increased to 0.062 $hr^{-1}$ for the remainder of the experiment. Results are shown in Table 3.

Table 3

| Age (hr) | Dilution Rate (hr$^{-1}$) | Polysaccharide (gm/litre) | Viscosity (CPS) |
|---|---|---|---|
| 190 | 0.042 | 25.9 | 18,500 |
| 310 | 0.042 | 28.0 | 18,000 |
| 478 | 0.042 | 28.6 | 28,500 |
| 598 | 0.042 | 29.5 | 30,000 |
| 646 | 0.062 | 28.3 | 26,500 |
| 694 | 0.062 | 28.6 | 26,500 |

**Claims**

1. A process for the production of polysaccharide by the continuous culture of polysaccharide producing bacteria of the genus *Xanthomonas* wherein the bacteria are grown in a single stage culture in a chemically defined culture medium, said medium having a growth limiting nutrilite and consisting of, in known ratio, a carbon source at a concentration of at least 10 gm litre$^{-1}$ (calculated as elemental carbon), a nitrogen source in the form of a single organic compound of known molecular structure and sources of phosphorus, sulphur, magnesium, potassium and other essential elements in the form of inorganic salts characterised in that the nitrogen source is the limiting nutrilite and is selected from the group consisting of glutamic acid, asparagine, a glutamate salt and an asparagine salt and further characterised in that the culture has a mean residence time in the culture vessel of 16⅔ hr or less.

2. A process according to claim 1 characterised in that the nitrogen source is glutamic acid or a glutamate salt.

3. A process according to either claim 1 or claim 2 characterised in that the total concentration of the nitrogen source in the culture medium is at least 0.7 gm litre$^{-1}$ (calculated as elemental nitrogen).

4. A process according to any one of claims 1 to 3 characterised in that the total concentration of the carbon source in the culture medium is between 15 and 24 gm litre$^{-1}$ (calculates as elemental carbon).

5. A process according to any one of claims 1 to 4 characterised in that the culture medium has a mean residence time, of culture medium in the culture vessel, of between 9 and 16⅔ hr.

**Patentansprüche**

1. Verfahren zur Herstellung von Polysacchariden durch kontinuierliches Kultivieren von Polysaccharide produzierenden Bakterien der Gattung Xanthomonas, wobei die Bakterien in einer Einstufen-Kultur in einem chemisch definierten Kulturmedium gezüchtet werden, das einen washstumsbegrenzenden Nährstoff enthält und in bekannten Verhältnissen aus einer Kohlenstoffquelle in einer Konzentration von mindestens 10 g·1$^{-1}$, bezogen auf elementaren Kohlenstoff, einer Stickstoffquelle in Form einer einzigen organischen Verbindung bekannter Molekularstruktur und Phosphor-, Schwefel-, Magnesium-, Kalium- und anderen wichtigen Elementquellen in Form von anorganischen Salzen besteht, dadurch gekennzeichnet, daß als wachstumsbegrenzender Nährstoff die Stickstoffquelle, ausgewählt unter Glutaminsäure, Asparagin, einem Glutamat und einem Asparaginsalz, verwendet wird und daß die Kultur eine mittlere Verweilzeit im Kulturgefäß von höchstens 16⅔ h hat.

2. Verfahren nach Anspruch 1, dadurch gekennzeichnet, daß als Stickstoffquelle Glutaminsäure oder ein Glutamat verwendet wird.

3. Verfahren nach Anspruch 1 oder 2, dadurch gekennzeichnet, daß eine Gesamtkonzentration an Stickstoffquelle im Kulturmedium von mindestens 0,7 g·1$^{-1}$, bezogen auf elementaren Stickstoff, verwendet wird.

4. Verfahren nach einem der Ansprüche 1 bis 3, dadurch gekennzeichnet, daß eine Gesamtkonzentration an Kohlenstoffquelle im Kulturmedium von 15 bis 24 g·1$^{-1}$, bezogen auf elementaren Kohlenstoff, verwendet wird.

5. Verfahren nach einem der Ansprüche 1 bis 4, dadurch gekennzeichnet, daß das Kulturmedium eine mittlere Verweilzeit im Kulturgefäß 9 bis 16⅔ h hat.

**Revendications**

1. Procédé pour produire du polysaccharide par la culture en continu d'une bactérie productrice de polysaccharide, du genre *Xanthomonas*, procédé dans lequel on fait croître les bactéries dans une culture en une seule étape dans un milieu de culture chimiquement défini, ledit milieu ayant un facteur nutritif ou nutrilite à rôle de limitation de croissance et consistant, selon un rapport connu, en une source de carbone présente en une concentration d'au moins 10 g/l (calculée en carbone élémentaire), une source d'azote sous forme d'un composé organique simple de structure moléculaire connue, et des sources de phosphore, de soufre, de magnésium, de potassium et d'autres éléments essentiels, sous forme de sels minéraux, procédé caractérisé en ce que la source d'azote constitue le facteur nutritif, ou nitrilite, à rôle de limitation et cette source est choisie dans l'ensemble constitué par l'acide glutamique, l'asparagine, un sel d'acide glutamique et un sel d'asparagine, et le procédé est en outre caractérisé en ce que la culture présente un temps moyen de séjour, dans le récipient de culture, égal ou inférieur à 16 heures ⅔.

2. Procédé selon la revendication 1, caractérisé en ce que la source d'azote est l'acide glutamique ou un sel d'acide glutamique.

3. Procédé selon l'une des revendications 1 ou 2, caractérisé en ce que la concentration totale de la source d'azote dans le milieu de culture est au moins égale à 0,7 g/l (concentration calculée en azote élémentaire).

4. Procédé selon l'une quelconque des revendications 1 à 3, caractérisé en ce que la concentration totale de la source de carbone dans le milieu de culture se situe entre 15 et 24 g/l (concentration calculée en carbone élémentaire).

5. Procédé selon l'une quelconque des revendications 1 à 4, caractérisé en ce que le milieu de culture présente un temps moyen de séjour du milieu de culture dans le récipient de culture compris entre 9 et 16 h ⅔.